# EUROPÄISCHE PATENTSCHRIFT

(11) **EP 1 508 318 B1**
(45) Veröffentlichungstag und Bekanntmachung des Hinweises auf die Patenterteilung: **04.10.2006**
(21) Anmeldenummer: 04017257.9
(22) Anmeldetag: 22.07.2004
(51) Int. Cl.: A61F 5/058, A61F 5/01

(54) **Orthopädische Schiene**
Orthopaedic splint.
Attelle orthopédique.

(30) Priorität: 12.08.2003 DE 10337332
(43) Veröffentlichungstag der Anmeldung: 23.02.2005
(73) Patentinhaber: Otto Bock HealthCare GmbH, 37115 Duderstadt (DE)
(72) Erfinder: Vollbrecht, Matthias, 37412 Herzberg (DE); Wagner, Helmut, 37115 Duderstadt (DE); Lidolt, Klaus, 37115 Duderstadt (DE); Mühlenberend, Andreas, 04103 Leipzig (DE)
(74) Vertreter: Lins, Edgar

(56) Entgegenhaltungen:
- WO-A-94/15555
- WO-A-99/59507
- DE-A1- 2 301 746
- DE-C- 520 959
- US-A- 2 260 216
- US-B1- 6 565 523

## Beschreibung

Die Erfindung betrifft eine orthopädische Schiene, die, insbesondere als Teil einer Orthese, zur Anlage an einem Körperteil bestimmt ist.

Für die Stützung von Körperteilen, insbesondere Extremitäten, mit einer orthopädischen Schiene ist es in vielen Fällen erforderlich, eine Anpassung der Schiene an das Körperteil vorzunehmen, um eine gewünschte Abstützung zu erzielen. Zu diesem Zweck ist es bekannt, eine orthopädische Schiene aus einem metallischen Flachmaterial herzustellen, das plastisch verformbar ist, um so die gewünschte Formgebung herbeizuführen. Nachteilig hieran ist die aufgrund der Verformbarkeit begrenzte Stabilität der Schiene, die beispielsweise nicht als Teil einer mit dem Körpergewicht belasteten Orthese benutzt werden kann. Dokument WO 99 59 507 A beschreibt beispielsweise eine derartige Vorrichtung.

Es ist daher bekannt, derartige Schienen, insbesondere als Bestandteil von Orthesen, speziell für den Anwendungsfall herzustellen, wodurch nicht unerhebliche Kosten verursacht werden. So werden beispielsweise im Prinzip gleiche Orthesen in unterschiedlichen Formen hergestellt, wenn sie zur Korrektur eines X-Beins oder zur Korrektur eines 0-Beins vorgesehen sind. Dadurch wird nicht nur die Herstellung sondern auch die Lagerhaltung aufwändiger.

Der Erfindung liegt die Aufgabe zugrunde, eine orthopädische Schiene der eingangs erwähnten Art so auszubilden, dass sie einfach herstellbar ist und einen geringeren Aufwand für die Lagerhaltung erfordert.

Zur Lösung dieser Aufgabe ist bei einer orthopädischen Schiene der eingangs erwähnten Art vorgesehen, dass die Schiene aus zwei etwa in einer Ebene angeordneten Rundstäben besteht, deren Enden beidseitig in Rundaufnahmen zweier Endstücke drehbar eingesteckt sind, dass beide Rundstäbe mit jeweils wenigstens einer Abwinkelung in der Ebene ausgebildet sind, dass die Rundaufnahmen eines Endstücks einen spitzen Winkel zueinander bilden und dass die Rundaufnahmen mit einem Übermaß gegenüber dem eingesteckten Ende so ausgebildet sind, dass in zwei Endstellungen unter Drehung der Rundstäbe unterschiedliche relative, aus der Ebene herausführende Winkel zwischen den Endstücken einstellbar sind.

Die erfindungsgemäße orthopädische Schiene ermöglicht somit eine aus der Ebene herausführende Winkelstellung der Endstücke zueinander, die vorzugsweise maximal 5° beträgt. Diese Abwinkelung ist in beiden Richtungen möglich, da die erfindungsgemäße Anordnung insbesondere zwei stabile Endstellungen ermöglicht, in denen die Orthese stabil belastbar ist. Aufgrund der beiden Endstellungen eignet sich die erfindungsgemäße orthopädische Schiene insbesondere für den Aufbau einer Orthese zur Korrektur einer X- oder O-Bein-Stellung, wobei für eine X-Bein-Korrektur die eine Endstellung und für eine O-Bein-Korrektur die andere Endstellung geeignet ist.

Die Funktionsweise der erfindungsgemäßen orthopädischen Schiene beruht darauf, dass die Rundstäbe in den Rundaufnahmen der Endstücke jeweils um eine Mittelachse der Rundaufnahmen drehbar gelagert sind, wobei die Drehung der Rundstäbe aufgrund der Abwinkelung zu einer Veränderung der Winkelstellung in der Rundaufnahme führt. Daher sind nur geringfügige Drehungen der Rundstäbe in der Rundaufnahme aufgrund des Übermaßes möglich, bis eine der Endstellungen erreicht ist. Die Drehung der Rundstäbe entspricht einer Einstellung eines relativen Winkels zwischen den Endstücken, d.h. einer Winkelstellung der Endstücke aus der (Ausgangs-) Ebene heraus. In der einen Endstellung erfolgt die Winkelstellung zur einen Seite, in der anderen Endstellung zur anderen Seite aus der Ebene heraus. Die jeweilige Einstellung der Schiene in einer der Endstellungen oder einer Zwischenstellung wird zweckmäßigerweise fixiert, vorzugsweise mittels einer Klemmeinrichtung.

In einer bevorzugten Ausführungsform, die insbesondere zur Anpassung an die Beinkontur geeignet ist, befinden sich die Abwinkelungen der Rundstäbe nahe einem der Endstücke. Somit wird der Winkel, der aus der Ebene herausführt, nahe einem der Endstücke ausgebildet, was insbesondere für eine Oberschenkelschiene zweckmäßig sein kann.

Die Abwinkelungen der beiden Rundstäbe sind vorzugsweise spiegelsymmetrisch zueinander ausgebildet. Dadurch wird sichergestellt, dass die durch die Winkelstellung erfolgte Formgebung in frontaler Ebene erfolgt und keine Verdrehung der Schiene in sich mit sich bringt.

Die Rundstäbe können aus Stabilitäts- und Gewichtsgründen vorzugsweise durch Rohre gebildet sein.

Wenn wenigstens eines der Endstücke der Schiene als Gelenkteil eines Drehgeienks ausgebildet ist, lässt sich die Schiene unproblematisch für eine Orthese mit einem Drehgelenk verwenden. Ein bevorzugter Anwendungsfall der erfindungsgemäßen Schiene besteht in der Ausbildung einer Orthese, die zur Korrektur einer X- oder O-Bein-Stellung dient. Hierzu sind vorzugsweise zwei Schienen über ein Drehgelenk miteinander verbunden.

Die Erfindung soll im Folgenden anhand eines in der Zeichnung dargestellten Ausführungsbeispiels näher erläutert werden. Es zeigen:
- Figur 1 -: eine Frontansicht eines Ausführungsbeispiels einer orthopädischen Schiene als Teil einer Orthese zur Korrektur der X- oder O-Bein-Stellung
- Figur 2 -: eine Rückansicht der Schiene gemäß Figur 1
- Figur 3a-c -: eine Seitenansicht der Orthese gemäß Figur 1 in einer ersten Formgebung, in einer zweiten Formgebung und schematisch mit beiden möglichen Formgebungen relativ zu einer Ausgangsebene.

Die in der Zeichnung dargestellte orthopädische Schiene besteht aus einem ersten Endstück 1, das zwei Rundaufnahmen aufweist, in die Enden eines ersten Rundstabs 2 und eines zweiten Rundstabs 3 eingesteckt sind. Die Rundaufnahmen bilden miteinander einen spitzen Winkel und sind symmetrisch zu einer Mittelachse des Endstücks 1 ausgerichtet, sodass die beiden Rundstäbe von dem Endstück 1 unter gleichen Winkeln zur Mittelachse mit zunächst geradlinigen Abschnitten voneinander weg zeigen. Die Rundstäbe 2, 3 gehen mit einer leichten Krümmung in nahezu parallel verlaufende Abschnitte über, bevor sie in der Nähe eines zweiten Endstücks 4 Abwinkelungen 5, 6 aufweisen, durch die die Rundstäbe 2, 3 nun mit zum zweiten Endstück 4 aufeinander zulaufenden Enden in das zweite Endstück 4 eingesteckt sind. Das zweite Endstück 4 steht in einem Winkel zur Mittelachse des ersten Endstücks 1, sodass die Enden der Rundstäbe 2, 3, die an sich symmetrisch zueinander ausgebildet sind, in unterschiedlichen Winkeln zu einer Mittenachse des zweiten Endstücks 4 stehen. Zur Anpassung an die Schrägstellung des zweiten Endstücks 4 ist der erste Rundstab 2 im Bereich seines zum zweiten Endstück 4 zeigenden Endes länger ausgebildet als der zweite Rundstab 3.

Das zweite Endstück 4 ist als Teil eines Drehgelenks 7 ausgebildet, durch das ein weiteres zweites Endstück 4' einer weiteren orthopädischen Schiene drehbar zum zweiten Endstück 4 der ersten Schiene angeordnet ist. In das Endstück 4' sind entsprechende Rundstäbe 2', 3' eingesteckt.

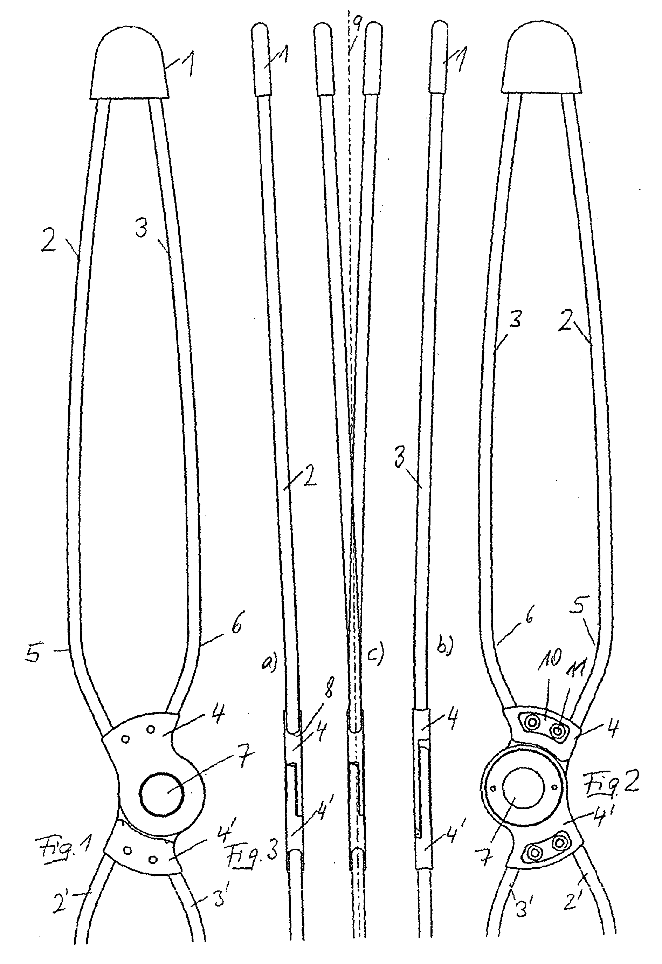

Figur 3a zeigt eine Seitenansicht auf die Schiene gemäß Figur 1, sodass der Rundstab 2 erkennbar ist. Erkennbar ist ferner eine Rundaufnahme 8 in dem zweiten Endstück 4 für das Ende des Rundstabs 2. In der in Figur 3a dargestellten Stellung sind die Rundstäbe 2, 3 so gedreht, dass sie in der Darstellung der Figur 3a nach links, in der Darstellung der Figur 3b (Rückansicht auf den Rundstab 3) nach rechts um einen geringen Winkel von ca. 3° abgebogen sind.

Figur 3c verdeutlicht eine Mittenebene 9, in der die Rundstäbe 2, 3 und die Endstücke 1, 4 im Wesentlichen angeordnet sind. Durch die erfindungsgemäße Ausbildung sind zwei stabile Endstellungen möglich, die in Figur 3c beide dargestellt sind und symmetrisch zur Ebene 9 geringfügige Winkelstellungen aufweisen. Die jeweilige Einstellung ist mit einer Klemmeinrichtung 10 fixierbar, die mittels eine Klemmung bewirkenden Schrauben 11 die Drehbarkeit der Rundstäbe 2, 3 unterbindet.

Die dargestellte Schiene ist Teil einer Orthese mit zwei Schienen mit dem Drehgelenk 7. Diese Orthese ist insbesondere zur Korrektur von X- bzw. O-Bein-Stellungen geeignet, wobei das Drehgelenk 7 in Höhe des Kniegelenks des Patienten angeordnet wird. Die in Figur 3c dargestellten Endstellungen verdeutlichen die Verstellmöglichkeit der Schiene zu ihrer Anpassung an die Beinkontur. Weiterhin kann auch eine Vorspannung in der Orthese erzeugt werden. Dadurch wird eine Entlastung erzielt, die für ein 0-Bein oder ein X-Bein verwendbar ist.

## Patentansprüche

1. Orthopädische Schiene, die, insbesondere als Teil einer Orthese, zur Anlage an einem Körperteil bestimmt ist, **dadurch gekennzeichnet, dass** die Schiene aus zwei etwa in einer Ebene (9) angeordneten Rundstäben (2, 3) besteht, deren Enden beidseitig in Rundaufnahmen (8) zweier Endstücke drehbar eingesteckt sind, dass beide Rundstäbe (2, 3) mit jeweils wenigstens einer Abwinkelung (5, 6) in der Ebene (9) ausgebildet sind, die Rundaufnahmen (8) eines Endstücks (1, 4) einen spitzen Winkel zueinander bilden und die Rundaufnahmen (8) mit einem Übermaß gegenüber dem eingesteckten Ende so ausgebildet sind, dass in zwei Endstellungen unter Drehung der Rundstäbe (2, 3) unterschiedliche relative, aus der Ebene (9) herausführende Winkel zwischen den Endstücken (1, 4) einstellbar sind.

2. Orthopädische Schiene nach Anspruch 1, **dadurch gekennzeichnet, dass** sich die Abwinkelungen (5, 6) der Rundstäbe (2, 3) nahe einem der Endstücke (1, 4) befinden.

3. Orthopädische Schiene nach Anspruch 1 oder 2, **dadurch gekennzeichnet, dass** die Abwinkelungen (5, 6) der beiden Rundstäbe (2, 3) spiegelsymmetrisch zueinander ausgebildet sind.

4. Orthopädische Schiene nach einem der Ansprüche 1 bis 3, **dadurch gekennzeichnet, dass** der relative Winkel zwischen den Endstücken (1, 4) einer Winkelstellung aus der Ebene (9) heraus von maximal 5 entspricht.

5. Orthopädische Schiene nach einem der Ansprüche 1 bis 4, **dadurch gekennzeichnet, dass** die Rundstäbe (2, 3) durch Rohre gebildet sind.

6. Orthopädische Schiene nach einem der Ansprüche 1 bis 5, **dadurch gekennzeichnet, dass** wenigstens eines der Endstücke (1, 4) als Gelenkteil eines Drehgelenks (7) ausgebildet ist.

7. Orthopädische Schiene nach einem der Ansprüche 1 bis 6, **dadurch gekennzeichnet, dass** sie Teil einer zur Korrektur einer X- oder O-Bein-Stellung dienenden Orthese ist.

8. Orthese mit zwei Schienen nach Anspruch 5 oder 6, die über ein Drehgelenk (7) miteinander verbunden sind.

## Claims

1. An orthopedic splint which, in particular as part of an orthosis, is intended to bear on a part of the body, **characterized in that** said splint consists of two round rods (2, 3) which are arranged approximately in a plane (9) and whose ends on both sides are inserted rotatably in round seats 8) of two endpieces, both round rods (2, 3) being designed with in each case at least one angled part (5, 6) in the plane {9), the round seats (8) of an endpiece (1, 4) forming an acute angle with respect to one another, and the round seats (8) being designed with an excess dimension relative to the inserted end so that, by rotating the round rods {2, 3), different relative angles extending out from the plane (9) can be set between the endpieces (1, 4) in two end settings.

2. The orthopedic splint as claimed in claim 1, wherein the angled parts (5, 6) of the round rods (2, 3) are situated near one of the endpieces (1, 4).

3. The orthopedic splint as claimed in claim 1 or 2, wherein the angled parts (5, 6) of the two round rods (2, 3) are designed mirror-symmetrically with respect to one another.

4. The orthopedic splint as claimed in one of claims 1 through 3, wherein the relative angle between the endpieces (1,4) corresponds to an angled setting out from the plane (9) of maximum 5°.

5. The orthopedic splint as claimed in one of claims 1 through 4, wherein the round rods (2, 3) are formed by tubes.

6. The orthopedic splint as claimed in one of claims 1 through 5, wherein at least one of the endpieces (1, 4) is designed as a hinge part of a pivot hinge (7).

7. The orthopedic splint as claimed in one of claims 1 through 6, wherein it is part of an orthosis serving for correction of genu valgum or genu varum.

8. An orthosis with two splints as claimed in claim 5 or 6, which splints are connected to one another via a pivot hinge (7).

## Revendications

1. Attelle orthopédique, destinée à être installée sur une partie du corps, notamment en tant que partie d'une orthèse, **caractérisée en ce que** l'attelle consiste en deux baguettes rondes (2, 3) agencées sensiblement dans un plan (9), **en ce que** des deux côtés les extrémités des baguettes sont insérées avec possibilité de rotation dans des logements ronds (8) de deux pièces d'extrémité, **en ce que** les deux baguettes rondes (2, 3) sont formées chacune avec au moins un coude (5, 6) dans le plan (9), les logements ronds (8) d'une pièce d'extrémité (1, 4) forment entre eux un angle aigu, et les logements ronds (8) sont formés avec un surdimensionnement par rapport à l'extrémité insérée, de sorte que des angles relatifs différents faisant sortir du plan, sont réglables entre les pièces d'extrémité (1, 4) dans deux positions extrêmes, par rotation des baguettes rondes (2, 3).

2. Attelle orthopédique selon la revendication 1, **caractérisée en ce que** les coudes (5, 6) des baguettes rondes (2, 3) se trouvent proches d'une des pièces d'extrémité (1, 4).

3. Attelle orthopédique selon la revendication 1 ou 2, **caractérisée en ce que** les coudes (5, 6) des deux baguettes rondes (2, 3) sont formés avec une symétrie de miroir l'un par rapport à l'autre.

4. Attelle orthopédique selon l'une des revendications 1 à 3, **caractérisée en ce que** l'angle relatif entre les pièces d'extrémité (1, 4) correspond à une position angulaire hors du plan (9) d'au maximum 5°.

5. Attelle orthopédique selon l'une des revendications 1 à 4, **caractérisée en ce que** les baguettes rondes (2, 3) sont formées par des tubes.

6. Attelle orthopédique selon l'une des revendications 1 à 5, **caractérisée en ce qu'**au moins une des pièces d'extrémité (1, 4) est formée en tant que pièce d'articulation d'une articulation tournante (7).

7. Attelle orthopédique selon l'une des revendications 1 à 6, **caractérisée en ce qu'**elle fait partie d'une orthèse servant pour la correction d'une position de jambes en X ou en O.

8. Orthèse avec deux attelles selon la revendication 5 ou 6, qui sont liées ensemble via une articulation tournante (7).
